# FASCICULE DE BREVET EUROPEEN

(11) **EP 2 029 237 B1**
(45) Date de publication et mention de la délivrance du brevet: **12.10.2016**
(21) Numéro de dépôt: 07765948.0
(22) Date de dépôt: 23.05.2007
(51) Int. Cl.: A61Q 5/04, A61K 8/365, A61K 8/362, A61K 8/73

(54) **PROCEDE DE DEFRISAGE DES FIBRES KERATINIQUES AVEC UN MOYEN DE CHAUFFAGE ET UN DERIVE D'ACIDE**
VERFAHREN ZUR GLÄTTUNG VON KERATINFASERN MIT ERWÄRMUNGSMITTEL UND EINEM SÄUREDERIVAT
METHOD FOR STRAIGHTENING KERATINOUS FIBERS USING HEATING MEANS AND AN ACID DERIVATIVE

(30) Priorité: 24.05.2006 FR 0651909; 20.06.2006 US 814904 P
(43) Date de publication de la demande: 04.03.2009
(60) Demande divisionnaire: 16166906.4
(73) Titulaire: L'Oréal, 75008 Paris (FR)
(72) Inventeur: MALLE, Gérard, F-77580 Villiers S/Morin (FR); BARBARAT, Philippe, F-92270 BOIS-COLOMBES (FR); PASINI, Isabelle, F-93600 Aulnay-sous-Bois (FR)
(74) Mandataire: Kromer, Christophe
(86) Numéro de dépôt international: PCT/FR2007/000872
(87) Numéro de publication internationale: WO 2007/135299

(56) Documents cités:
- EP-A- 1 532 963
- US-A- 5 957 140
- US-A- 6 125 856
- US-A1- 2003 143 173
- US-A1- 2006 024 257
- US-A1- 2006 104 928

## Description

L'invention a pour objet un procédé de défrisage des cheveux avec un moyen de chauffage et au moins un dérivé d'alpha-hydroxy-acide.

Le procédé de défrisage selon l'invention est réalisé sans utiliser d'agent réducteur, ni d'agent de lanthionisation. Il ne comprend aucune étape de réduction, ni de lanthionisation.

Le terme *"défrisage"* englobe, selon l'invention, le défrisage, le lissage ou le décrêpage de cheveux caucasiens ou africains. Le terme *« défriser »* englobe, selon l'invention, défriser, lisser ou décrêper des cheveux caucasiens ou africains.

Par *« moyen de chauffage»,* on entend tout moyen permettant de chauffer les fibres kératiniques à une température d'au moins 110°C tels que les fers chauffants, par exemple les fers plats ou ronds, les générateurs de micro-ondes, les sources de rayonnement infra-rouge.

Deux techniques sont utilisées pour obtenir une déformation permanente des cheveux. Elles sont basées sur une rupture des liaisons covalentes disulfures présentes dans la kératine (cystine) :
- la première consiste, dans un premier temps à réaliser cette ouverture des liaisons disulfures à l'aide d'une composition contenant un agent réducteur, puis après avoir, de préférence, rincé les cheveux, à reconstituer dans un second temps les dites liaisons disulfures en appliquant sur les cheveux préalablement mis sous tension par des bigoudis ou autres ou mis en forme ou lissés par d'autres moyens, une composition oxydante encore appelée fixateur, de façon à donner à la chevelure la forme recherchée.
- Cette technique permet indifféremment de réaliser, soit l'ondulation des cheveux, soit leur défrisage ou leur décrêpage ou leur lissage.
- La seconde consiste à effectuer une opération dite de lanthionisation, à l'aide d'une composition contenant une base appartenant à la famille des hydroxydes. Elle conduit à remplacer des liaisons disulfures (-CH2-S-S-CH2-) par des liaisons lanthionines (-CH2-S-CH2-). Cette opération de lanthionisation fait intervenir deux réactions chimiques consécutives :
   ▪ La première réaction consiste en une béta-élimination sur la cystine provoquée par un ion hydroxyde, conduisant à la rupture de cette liaison et à la formation de déhydro-alanine. 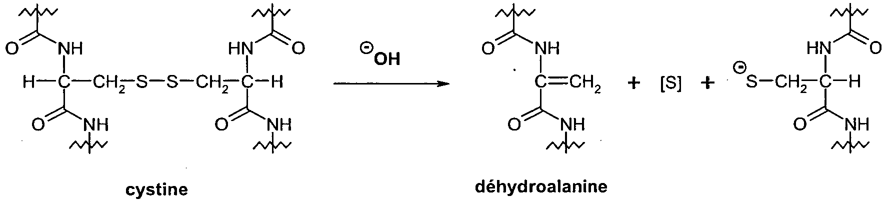
   ▪ La deuxième réaction est une réaction de la déhydro-alanine avec un groupe thiol. En effet, la double-liaison de la déhydro-alanine formée est une double-liaison réactive. Elle peut réagir avec le groupe thiol du résidu cystéine qui a été libéré pour former une nouvelle liaison appelée pont ou liaison ou résidu lanthionine.

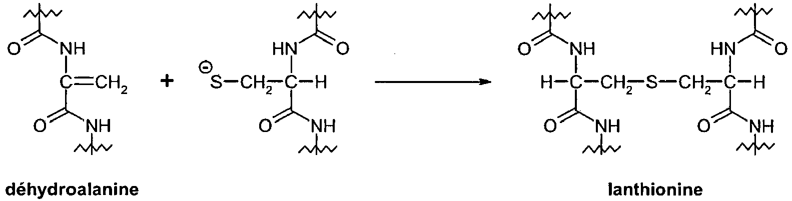

Par rapport à la première technique mettant en oeuvre un agent réducteur, cette technique de lanthionisation ne nécessite pas d'étape de fixation, puisque la formation des ponts lanthionine est irréversible. Elle s'effectue donc en une seule étape et permet indifféremment de réaliser soit l'ondulation des cheveux, soit leur défrisage ou leur décrêpage ou leur lissage. Cependant, elle est principalement utilisée pour le défrisage des cheveux naturellement crépus.

Pour la première technique, les compositions réductrices généralement utilisées pour la première étape d'une opération de permanente ou de défrisage contiennent à titre d'agent réducteur des thiols ou des sulfites ou des bisulfites. Ces agents sont généralement employés en milieu essentiellement aqueux à des concentrations comprises entre 0,5 et 1M pour obtenir une bonne ouverture des liaisons disulfures. Parmi les thiols, ceux couramment utilisés sont l'acide thioglycolique, la cystéamine, le monothioglycolate de glycérol, l'acide thiolactique et la cystéine. L'acide thioglycolique est particulièrement efficace pour réduire les liaisons disulfures de la kératine à pH alcalin, notamment sous forme de thioglycolate d'ammonium, et constitue le produit le plus utilisé en permanente (" hair waving "). On a toutefois constaté que l'acide thioglycolique doit être utilisé en milieu suffisamment basique (en pratique à pH compris entre 8,5 et 9,5) si on veut obtenir une frisure satisfaisante en intensité. Outre l'inconvénient de dégager une odeur désagréable nécessitant l'usage de parfums plus ou moins efficaces pour masquer les odeurs, l'utilisation d'un thiol à pH alcalin conduit également à des dégradations de la fibre et tout particulièrement à l'altération des colorations artificielles.

Les sulfites ou bisulfites sont principalement utilisés pour le défrisage. Ils possèdent des désavantages similaires aux thiols avec une efficacité moindre.

Les thiols et les sulfites (ou bisulfites) présentent en outre l'inconvénient d'avoir une mauvaise stabilité en solution aqueuse.

D'une façon générale, la durabilité des effets de déformations obtenus avec les thiols et les sulfites par réduction des disulfures puis fixation est jugée très inférieure à celle pouvant être obtenue par la technique de lanthionisation.

Pour la deuxième technique, les compositions généralement utilisées pour effectuer la lanthionisation contiennent à titre de base un hydroxyde tel que l'hydroxyde de sodium, l'hydroxyde de guanidinium et l'hydroxyde de lithium. Ces actifs de lanthionisation, permettant d'ouvrir les liaisons disulfures par un mécanisme de béta-élimination, sont généralement employés en émulsion eau-huile à des concentrations comprises entre 0,4 et 0,6M, en les laissant agir généralement 10 à 15 minutes à température ambiante. L'hydroxyde de sodium reste l'agent le plus utilisé. L'hydroxyde de guanidinium est maintenant le composé préféré pour de nombreuses compositions. Ces deux hydroxydes, de sodium et de guanidinium, sont les deux agents principaux utilisés pour le défrisage ou le décrêpage des cheveux naturellement crépus. Ils possèdent plusieurs avantages par rapport au thioglycolate d'ammonium et aux sulfites, en particulier une absence d'odeur désagréable, le fait qu'une seule étape de mise en oeuvre est requise (durée du traitement plus courte), et une durabilité et une efficacité beaucoup plus importante de la déformation du cheveu.
Cependant, ces hydroxydes présentent l'inconvénient majeur d'être caustiques. Cette causticité affecte le cuir chevelu en provoquant des irritations parfois sévères. On peut y remédier partiellement par l'application préalable sur le scalp d'une crème protectrice grasse souvent appelée " base " ou " crème base ", le mot " base " ici utilisé n'ayant pas la signification d'agent basique au sens chimique. Lorsque la crème protectrice est associée à l'hydroxyde en une seule composition, celle-ci est généralement appelée " no-base ", par opposition à l'appellation ci-dessus. Cette technologie " no-base " est préférée.

La causticité des hydroxydes affecte également l'état du cheveu en le rendant d'une part rêche au toucher et d'autre part beaucoup plus fragile, cette fragilité pouvant aller jusqu'à l'effritement voire la rupture ou même la dissolution des cheveux si le traitement est prolongé. Les hydroxydes provoquent dans certains cas également des décolorations de la couleur naturelle du cheveu.

Les formules contenant de l'hydroxyde de sodium sont généralement dénommées en anglais " lye relaxers" et celles n'en contenant pas sont dénommées " no-lye relaxers ".

Les principales formules défrisantes dites " no-lye " mettent en oeuvre l'hydroxyde de guanidinium. L'hydroxyde de guanidinium étant instable, celui-ci est généré extemporanément par le mélange de carbonate de guanidine et d'une source d'hydroxyde très peu soluble telle que l'hydroxyde de calcium. La réaction entre ces deux composés conduit à la formation d'hydroxyde de guanidinium et de carbonate de calcium qui précipite dans la composition. La présence de ce précipité rend le rinçage final des cheveux beaucoup plus difficile et laisse sur les cheveux et le scalp des particules minérales qui lui donnent un toucher rêche et une apparence inesthétique ressemblant aux pellicules. Le succès récent de l'hydroxyde de guanidinium (" no-lye ") face à l'hydroxyde de sodium (" lye ") semble provenir d'une meilleure efficacité de défrisage et d'une meilleure tolérance cutanée. Cependant ces technologies mettant en oeuvre des bases de la famille des hydroxydes demeurent très agressives pour le cheveu et le cuir chevelu et demandent un contrôle très strict de la durée d'application pour éviter les irritations trop fortes et l'altération des cheveux pouvant aller jusqu'à la cassure. Cette agressivité provenant de la causticité des hydroxydes justifie que ces compositions pour la lanthionisation du cheveu ne soient pas utilisées pour la permanente (" hair waving ") mais réservées au défrisage (" hair straightening " ou " hair relaxing ").

De plus, les hydroxydes sont connus pour être de bons agents d'hydrolyse des fonctions amides (cf par exemple March's Advanced Organic Chemistry, 5ed., Wiley Interscience, New York, " Hydrolysis of Amides " pages 474 et suivantes) qui conduisent donc à la rupture des liaisons peptidiques par attaque nucléophile directe. Il est ainsi probable que les altérations constatées au niveau des cheveux et des matières kératiniques au sens large soient en grande partie dues à une hydrolyse partielle des liaisons amides de la kératine.

Il existe donc un réel besoin pour le défrisage de compositions nettement moins agressives pour le cheveu.

Diverses études ont été conduites en vue de remédier à la fois aux inconvénients des agents réducteurs (première technique) et/ou des hydroxydes (deuxième technique).

Ainsi pour remplacer l'acide thioglycolique, de nombreux agents réducteurs ont été proposés, mais l'acide thioglycolique sous sa forme de thioglycolate d'ammonium reste à la fois le composé de référence et le plus largement utilisé dans les formulations cosmétiques, aussi bien pour la mise en forme que pour le lissage.

Il a également été proposé dans de très nombreux brevets d'associer des agents réducteurs habituels (thiols ou sulfites ou bisulfites) avec de l'urée ou des alkylurées pour diminuer l'irritation et les dommages causés aux cheveux aussi bien pour la mise en forme que pour le défrisage. On citera par exemple :
- la demande CA 1315204 qui décrit une composition contenant du thioglycolate d'ammonium (5,5-11,5%) et de l'urée ou une monoalkylurée (1-3%) pour la mise en forme des cheveux,
- la demande US 3847165 qui décrit une composition contenant du thioglycolate d'ammonium (1,2-1,4M) et de l'urée (2,0-2,7M) pour la mise en forme des cheveux à un pH acide,
- la demande NL 6410355 qui décrit une composition contenant un sulfite (0,8-1,5M) et de l'urée (0,6-3,0M) pour la mise en forme et le défrisage des cheveux,
- la demande JP 2000/229819 qui décrit une composition contenant un sulfite ou bisulfite (0,5-15%), de l'urée (0,5-15%) et un alcool (éthanol et/ou isopropanol, 1-30%) pour la mise en forme et le défrisage des cheveux,

Il a également été proposé dans de très nombreux brevets d'associer des hydroxydes, servant d'actif de lanthionisation, avec certains additifs servant généralement à protéger les cheveux. On citera, à titre d'exemple:
- la demande WO2002/003937, qui décrit une composition contenant des monosaccharides en C3-C5,
- la demande WO2001/064171, qui décrit une composition contenant des agents complexants,
- le brevet US5641477, qui décrit une composition contenant un hydrolysat d'amidon hydrogéné,
- la demande WO02085317, qui décrit une composition contenant des nucléophiles organiques qui réagissent lors de la deuxième étape avec la déhydro-alanine formée avec des hydroxydes, pour conduire à de nouveaux pontages.

Si toutes ces propositions conduisent à des améliorations plus ou moins marquées, elles ne permettent pas de diminuer de façon suffisante les dégâts liés à la causticité même des hydroxydes.

Comme indiqué précédemment, l'utilisation d'agents réducteurs conduit à une durabilité médiocre pour le défrisage ou le décrêpage et l'emploi d'hydroxydes, de part leur causticité, limite leur utilisation au domaine du défrisage.

La publication Journal of Textile Institute, 1979, n°7, 318-320 décrit un appareil pour défriser une peau de mouton à l'aide d'un fer à repasser et avec l'application d'acide glyoxylique.

La demande JP-A-2001-213741 décrit un procédé de traitement des cheveux comprenant l'application sur les cheveux d'un agent de traitement contenant un acide, un polymère cationique, un solvant et un aminoacide et/ou un hydrolysat de protéine.

Après d'importantes études, on vient maintenant de découvrir, de façon tout à fait surprenante et inattendue, que l'on pouvait défriser durablement le cheveu en associant l'action d'un dérivé d'alpha-hydroxy- et/ou cétoacide et d'un moyen de chauffage à une température supérieure à 110°C. On obtient ainsi d'excellents résultats en terme de défrisage, de propriétés cosmétiques des cheveux et d'intégrité de la fibre.

Sans être liée par la théorie, la Demanderesse pense qu'il existe une action conjointe, sur les cheveux, d'un dérivé d'alpha-hydroxy-acide et d'un moyen de chauffage, qui permet de les défriser de façon efficace et durable.

La Demanderesse a trouvé qu'il était possible de remédier aux inconvénients de l'art antérieur et de remplir les objectifs précités en mettant en oeuvre un procédé de défrisage des cheveux comprenant :
- une étape d'application sur les fibres kératiniques d'une composition de défrisage contenant au moins un dérivé d'alpha-hydroxy-acide de formule (I) décrit ci-après, le pH de cette composition étant inférieur ou égal à 7,
- puis une étape d'élévation de la température des cheveux, à l'aide d'un moyen de chauffage, à une température comprise entre 140 et 250°C,par application d'un fer faite en mouvement continu de la racine à la pointe, en un ou plusieurs passages,
le procédé de défrisage étant réalisé sans étape de réduction , ni de lanthionisation

Ainsi, l'invention a pour objet un procédé de défrisage des cheveux comprenant :
- une étape d'application sur les cheveux d'une composition de défrisage contenant au moins un dérivé d'alpha-hydroxy-acide, le pH de cette composition étant inférieur ou égal à 7
- puis une étape d'élévation de la température des cheveux, à l'aide d'un moyen de chauffage, à une température comprise entre 140 et 250°C, par application d'un fer faite en mouvement continu de la racine à la pointe, en un ou plusieurs passages,
le procédé de défrisage étant réalisé sans étape de réduction , ni de lanthionisation

Avantageusement, on élève la température à l'aide du moyen de chauffage à une température comprise entre 140°C et 220°C.

De préférence, ladite composition est appliquée sur des cheveux humides.

On peut également avantageusement intercaler entre l'étape d'application de la composition et l'étape d'élévation de température, une étape destinée à éliminer l'excédant de la composition, par exemple au moyen d'une serviette.

### Définition des dérivés d'α-hydroxyacides de formule générale (I) :

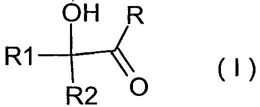

R1 = H, OH, NH2, CH2-COOH, alkyle C1-C4 linéaire ou ramifié
R2 = H, COOH, CHOH-COOH, CF3, CH=CH2, NHCONH2, alkyle C1-C8 linéaire ou ramifié ou cyclique éventuellement substitué par un radical choisi parmi OH, Cl, NH2, COOH, CF3 ou SCH3 ;
phényle ou benzyle éventuellement substitués par 1 radical OH ou OCH3 ; ou encore le radical 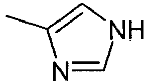
R1 et R2 peuvent également former ensemble un radical oxo (=O) ou un cycle cyclopropyle ou cyclobutyle ou hydroxycyclobutyle ou cyclopentyle ou cyclohexyle avec l'atome de carbone qui les porte ou encore un radical 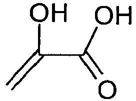
Lorsque R1 = H, R2 peut également représenter le radical (CHOH)2CH2OH ou (CHOH)3CH2OH
R = OH ou NR3R4 avec R3, R4 = H ou alkyle C1-C4 linéaire ou ramifié éventuellement substitué par 1 ou 2 radicaux OH

Et leurs stéréoisomères et sels organiques ou minéraux et solvates

### Composés de formule (I) préférés :

L'acide glycolique
L'acide oxalique
L'acide lactique
L'acide 1-hydroxy-1-cyclopropane carboxylique
L'acide 2-hydroxy-3-buténoïque
L'acide 2- hydroxy isobutyrique
L'acide 2-hydroxy-n-butyrique
L'isosérine
L'acide glycérique
L'acide 2- hydroxy -3 - méthyl butyrique
L'acide 2-hydroxy-2-méthyl butyrique
L'acide 2-hydroxyvalérique
L'acide 4-amino-2-hydroxybutyrique
L'acide 1-hydroxycyclohexanecarboxylique
L'acide dihydroxyfumarique
L'acide citramalique
L'acide tartrique
L'acide citrique
L'acide 2- hydroxy-4-(méthylthio)butyrique
L'acide mandélique
L'acide 2- hydroxy-3-méthyl valérique
La glyoxylurée
L'acide ß-imidazole lactique
L'acide 2- trifluorométhyl-2-hydroxypropionique
L'acide hexahydromandélique
L'acide 2-hydroxyoctanoïque
L'acide arabique
L'acide 3-phényllactique
L'hydroxyphényl glycine
L'acide 3-hydroxy mandélique
L'acide 4-hydroxy mandélique
L'acide 2-hydroxynonanoïque
l'acide L - arginique
L'acide 3-méthoxy mandélique
L'acide 4-méthoxy mandélique
L'acide 3-(4-hydroxyphényl)lactique
L'acide tartronique
L'acide tartrique
L'acide ß-chlorolactique
L'acide 1-cyclopentanol-1-carboxylique
L'acide 1,2-dihydroxycyclobutane carboxylique
L'acide 2-éthyl-2-hydroxy butyrique
L'acide α-hydroxyisocaproïque
L'acide α-hydroxycaproïque
L'acide 2-hydroxy-3,3-diméthylbutyrique
L'acide malique
L'acide hydroxytartronique
L'acide gluconique
Le lactamide
Le N-méthyl-lactamide
Le N-éthyl-lactamide
Le N,N-diméthyl-lactamide
Le N- 2-hydroxyéthyl-lactamide

Et leurs stéréoisomères et sels organiques ou minéraux et solvates

### Composés de formule_(I) particulièrement préférés :

L'acide glycolique
L'acide oxalique
L'acide L lactique
L'acide DL lactique
L'acide D lactique
L'acide malique
L'acide tartrique
L'acide DL - glycérique
L'acide arabique
L'acide gluconique
L'acide hydroxytartronique
Le lactamide
Le N-méthyl-lactamide
Le N-éthyl-lactamide
Le N- 2-hydroxyéthyl-lactamide

### Concentrations d'utilisation

La concentration molaire d'utilisation est avantageusement comprise entre 2 et 8M, plus avantageusement entre 4 et 8M

Les compositions selon l'invention se présentent soit sous la forme d'une solution aqueuse, soit sous forme d'une crème épaissie de façon à maintenir les cheveux aussi raides que possible. On réalise ces crèmes, sous forme d'émulsions « lourdes ». Ces compositions renferment au moins un dérivé d'alpha-hydroxyacide de formule (I).

Avantageusement, les compositions de l'invention renferment le dérivé d'alpha-hydroxy-acide comme seul actif de défrisage.

Dans le but d'améliorer les propriétés cosmétiques des cheveux ou encore d'atténuer ou d'éviter leur dégradation, la composition utilisée selon l'invention peut également comprendre un ou plusieurs actifs cosmétiques supplémentaires.

Généralement, le ou lesdits actifs cosmétiques supplémentaires représentent de 0,01 à 30%, de préférence de 0,1 à 10%, en poids du poids total de la composition cosmétique.

Généralement, la composition appliquée sur les cheveux est appliquée à hauteur de 0,05 à 20 g, de préférence de 0,1 à 10 g de composition par gramme de fibre kératinique sèche.

Après application de la composition, et avant l'élévation de la température des cheveux au moyen d'un moyen de chauffage, on peut laisser poser ladite composition, généralement pendant 30 secondes à 60 minutes, de préférence 5 à 45 minutes.

Le procédé selon l'invention comprend, après l'étape d'application de la composition, une étape d'élévation de la température des cheveux, au moyen d'un moyen de chauffage, à une température comprise entre 140°C et 250°C.

On utilise un fer comme moyen de chauffage.

Au sens de la présente invention, on entend par « fer » un dispositif de chauffage des cheveux mettant en contact lesdits cheveux et le dispositif de chauffage.

L'extrémité du fer venant en contact avec les cheveux présente généralement deux surfaces planes. Ces deux surfaces planes peuvent être métalliques. Elles peuvent être lisses ou crantées.

A titre d'exemple de fers utilisables dans le procédé selon l'invention, on peut citer tous types de fer plats et, en particulier, de manière non limitative, ceux décrits dans les brevets US 5 957 140, et US 5 046 516.

L'application du fer peut être effectuée par touches séparées successives de quelques secondes, ou par déplacement ou glissement progressif le long des mèches.

De préférence, l'application du fer dans le procédé selon l'invention se fait en mouvement continu de la racine à la pointe, en un ou plusieurs passages.

Le procédé selon l'invention peut également comprendre une étape supplémentaire de pré-séchage partiel des cheveux avant l'étape d'élévation de la température, de manière à éviter d'importants dégagements de vapeurs qui pourraient brûler les mains du coiffeur et le cuir chevelu de la personne. Cette étape de pré-séchage peut se faire par exemple au moyen d'un séchoir, d'un casque, ou bien encore par séchage libre.

L'invention pourra être mieux comprise à l'aide des exemples non limitatifs qui suivent et qui constituent des modes de réalisation préférentiels des compositions selon l'invention.

### EXEMPLE 1:

On réalise une composition de défrisage simplifiée contenant l'acide DL-lactique, à une concentration de 8M dans l'eau, en tant qu'actif de défrisage. On applique cette composition sur des cheveux africains naturellement frisés pendant 15 minutes à une température de 40°C puis on essuie rapidement les cheveux avec une serviette.

On procède ensuite à un lissage mèche à mèche de la chevelure à l'aide d'un fer plat chauffé à 180°C pendant 10 à 15 secondes. Les cheveux sont défrisés efficacement et doux au toucher.

### EXEMPLE 2:

On réalise une composition de défrisage simplifiée contenant l'acide L-lactique, à une concentration de 4M dans l'eau, en tant qu'actif de défrisage. On applique cette composition sur des cheveux africains naturellement frisés pendant 25 minutes à une température de 40°C puis on essuie rapidement les cheveux avec une serviette.

On procède ensuite à un lissage mèche à mèche de la chevelure à l'aide d'un fer plat chauffé à 180°C pendant 10 à 15 secondes. Les cheveux sont défrisés efficacement et doux au toucher.

## Revendications

1. Procédé de défrisage des cheveux comprenant :
- une étape d'application sur les cheveux d'une composition de défrisage contenant au moins un dérivé d'alpha-hydroxy- acide, le pH de cette composition étant inférieur ou égal à 7,
- puis une étape d'élévation de la température des cheveux, à l'aide d'un moyen de chauffage, à une température comprise entre 140 et 250°C, par application d'un fer faite en mouvement continu de la racine à la pointe, en un ou plusieurs passages,
le procédé de défrisage étant réalisé sans étape de réduction , ni de lanthionisation,
le dérivé d'alpha hydroxyacide étant choisi parmi les composés de formule (I) : 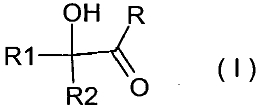 dans laquelle :
R1 = H, OH, NH2, CH2-COOH, alkyle C1-C4 linéaire ou ramifié
R2 = H, COOH, CHOH-COOH, CF3, CH=CH2, NHCONH2, alkyle C1-C8 linéaire ou ramifié ou cyclique éventuellement substitué par un radical choisi parmi OH, C1, NH2, COOH, CF3 ou SCH3 ;
phényle ou benzyle éventuellement substitués par 1 radical OH ou OCH3 ; ou encore le radical 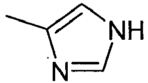
R1 et R2 peuvent également former ensemble un radical oxo (=O) ou un cycle cyclopropyle ou cyclobutyle ou hydroxycyclobutyle ou cyclopentyle ou cyclohexyle avec l'atome de carbone qui les porte ou encore un radical 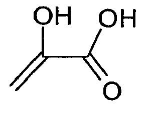
lorsque R1 = H, R2 peut également représenter le radical (CHOH)2CH2OH ou (CHOH)3CH2OH
R = OH ou NR3R4 avec R3, R4 = H ou alkyle C1-C4 linéaire ou ramifié éventuellement substitué par 1 ou 2 radicaux OH
et leurs stéréoisomères et sels organiques ou minéraux et solvates.

2. Procédé selon la revendication 1, **caractérisé par le fait qu'**on on élève la température à l'aide du moyen de chauffage à une température comprise entre 140°C et 220°C.

3. Procédé selon l'une quelconque des revendications précédentes, **caractérisé par le fait que** la composition est appliquée sur des cheveux humides.

4. Procédé selon l'une quelconque des revendications précédentes, **caractérisé par le fait que** les cheveux sont partiellement préséchés.

5. Procédé selon l'une quelconque des revendications précédentes, **caractérisé par le fait que** la concentration molaire en dérivé d'alpha-hydroxy-acide est comprise entre 2 et 8 M.

6. Procédé selon l'une quelconque des revendications précédentes, **caractérisé par le fait que** le composé de formule (I) est choisi parmi:
L'acide glycolique
L'acide oxalique
L'acide lactique
L'acide 1 -hydroxy-1-cyclopropane carboxylique
L'acide 2- hydroxy - 3 - buténoïque
L'acide 2- hydroxy isobutyrique
L'acide 2-hydroxy-n-butyrique
L'isosérine
L'acide glycérique
L'acide 2- hydroxy -3 - méthyl butyrique
L'acide 2-hydroxy-2-méthyl butyrique
L'acide 2-hydroxyvalérique
L'acide 4-amino-2-hydroxybutyrique
L'acide 1-hydroxycyclohexanecarboxylique
L'acide dihydroxyfumarique
L'acide citramalique
L'acide tartrique
L'acide citrique
L'acide 2- hydroxy-4-(méthylthio)butyrique
L'acide mandélique
L'acide 2- hydroxy-3-méthyl valérique
La glyoxylurée
L'acide ß-imidazole lactique
L'acide 2- trifluorométhyl-2-hydroxypropionique
L'acide hexahydromandélique
L'acide 2-hydroxyoctanoïque
L'acide arabique
L'acide 3-phényllactique
L'hydroxyphényl glycine
L'acide 3-hydroxy mandélique
L'acide 4-hydroxy mandélique
L'acide 2-hydroxynonanoïque
L'acide 3-méthoxy mandélique
L'acide 4-méthoxy mandélique
L'acide 3-(4-hydroxyphényl)lactique
L'acide tartronique
L'acide ß-chlorolactique
L'acide 1-cyclopentanol-1-carboxylique
L'acide 1,2-dihydroxycyclobutane carboxylique
L'acide 2-éthyl-2-hydroxy butyrique
L'acide α-hydroxyisocaproïque
L'acide α-hydroxycaproïque
L'acide 2-hydroxy-3,3-diméthylbutyrique L'acide malique
L'acide hydroxytartronique
L'acide gluconique
Le lactamide
Le N-méthyl-lactamide
Le N-éthyl-lactamide
Le N,N-diméthyl-lactamide
Le N- 2-hydroxyéthyl-lactamide
Et leurs stéréoisomères et sels organiques ou minéraux et solvates

7. Procédé selon l'une quelconque des revendications précédentes, **caractérisé par le fait que** le composé de formule (I) est choisi parmi:
L'acide glycolique
L'acide oxalique
L'acide lactique
L'acide 2- hydroxy isobutyrique
L'acide 2-hydroxy-n-butyrique
L'acide glycérique
L'acide 2- hydroxy -3 - méthyl butyrique
L'acide 2-hydroxy-2-méthyl butyrique
L'acide 2-hydroxyvalérique
L'acide 4-amino-2-hydroxybutyrique
L'acide citramalique
L'acide tartrique
L'acide citrique
L'acide 2- hydroxy-3-méthyl valérique
L'acide 2-hydroxyoctanoïque
L'acide arabique
L'acide 2-hydroxynonanoïque
L'acide tartronique
L'acide 2-éthyl-2-hydroxy butyrique
L'acide α-hydroxyisocaproïque
L'acide α-hydroxycaproïque
L'acide 2-hydroxy-3,3-diméthylbutyrique L'acide malique
L'acide hydroxytartronique
L'acide gluconique
et leurs stéréoisomères et sels organiques ou minéraux et solvates.

8. Procédé selon selon l'une quelconque des revendications précédentes, **caractérisé par le fait que** le composés de formule (I) est choisi parmi:
L'acide glycolique
L'acide oxalique
L'acide lactique
L'acide 2-hydroxy isobutyrique
L'acide malique
L'acide tartrique
L'acide 4-amino-2-hydroxybutyrique
L'acide citrique
L'acide gluconique
L'acide tartronique
L'acide α-hydroxycaproïque
et leurs stéréoisomères et sels organiques ou minéraux et solvates.

## Patentansprüche

1. Verfahren zur Haarentkräuselung, das Folgendes umfasst:
- einen Schritt, in welchem eine Entkräuselungszusammensetzung auf das Haar aufgebracht wird, die mindestens ein alpha-Hydroxysäurederivat enthält, wobei der pH-Wert dieser Zusammensetzung höchstens 7 beträgt,
- anschließend einen Schritt, in welchem die Temperatur des Haars mit Hilfe eines Erhitzungsmittels auf eine Temperatur im Bereich von 140 bis 250 °C erhöht wird, durch Aufbringen eines Glätteisens, wobei dies in einer kontinuierlichen Bewegung von der Wurzel zur Spitze erfolgt, in einem oder mehreren Durchgängen,
wobei das Entkräuselungsverfahren ohne Reduktionsschritt und Lanthionineinsatz durchgeführt wird,
wobei das alpha-Hydroxysäurederivat aus den Verbindungen der folgenden Formel (I) ausgewählt ist: 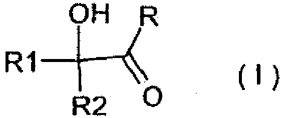 wobei:
R1 = H, OH, NH2, CH2-COOH, geradkettiges oder verzweigtes C1-C4-Alkyl ist
R2 = H, COOH, CHOH-COOH, CF3, CH=CH2, NHCONH2, geradkettiges oder verzweigtes oder zyklisches C1-C8-Alkyl, das möglicherweise mit einem Rest substituiert ist, welcher aus OH, Cl, NH2, COOH, CF3 oder SCH3 ausgewählt ist;
Phenyl oder Benzyl, welches möglicherweise mit einem OH- oder OCH3-Rest substituiert ist; oder auch dem Rest 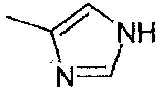 ist
wobei R1 und R2 auch gemeinsam einen oxorest (=O) oder, mit dem Kohlenstoffatom, an welches sie gebunden sind, einen Cyclopropyl- oder Cyclobutyl- oder Hydroxycyclobutyl- oder Cyclopentyl- oder Cyclohexylring bilden können, oder aber einen Rest 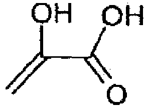
wenn R1 = H ist, kann R2 auch für den Rest (CHOH)2CH2OH oder (CHOH)3CH2OH stehen
R = OH oder NR3R4 mit R3, R4 = H oder geradkettiges oder verzweigtes C1-C4-Alkyl, das möglicherweise mit 1 oder 2 OH-Resten substituiert ist
und deren Stereoisomeren und organischen oder mineralischen Salzen sowie Solvaten.

2. Verfahren nach Anspruch 1, **dadurch gekennzeichnet, dass** die Temperatur mit Hilfe eines Erhitzungsmittels auf eine Temperatur im Bereich von 140 °C bis 220 °C erhöht wird.

3. Verfahren nach einem beliebigen der vorhergehenden Ansprüche, **dadurch gekennzeichnet, dass** die Zusammensetzung auf das feuchte Haar aufgebracht wird.

4. Verfahren nach einem beliebigen der vorhergehenden Ansprüche, **dadurch gekennzeichnet, dass** das Haar teilweise vorgetrocknet wird.

5. Verfahren nach einem beliebigen der vorhergehenden Ansprüche, **dadurch gekennzeichnet, dass** die molare Konzentration an alpha-Hydroxysäurederivat im Bereich von 2 bis 8 M liegt.

6. Verfahren nach einem beliebigen der vorhergehenden Ansprüche, **dadurch gekennzeichnet, dass** die Verbindung nach Formel (I) aus den folgenden ausgewählt ist:
Glycolsäure
Oxalsäure
Milchsäure
1-Hydroxy-1-cyclopropancarbonsäure
2-Hydroxy-3-butensäure
2-Hydroxyisobuttersäure
2-Hydroxy-n-buttersäure
Isoserin
Glycerinsäure
2-Hydroxy-3-methylbuttersäure
2-Hydroxy-2-methylbuttersäure
2-Hydroxyvaleriansäure
4-Amino-2-hydroxybuttersäure
1-Hydroxycyclohexancarbonsäure
Dihydroxyfumarsäure
2-Methyläpfelsäure
Weinsäure
Citronensäure
2-Hydroxy-4-(methylthio)buttersäure
Mandelsäure
2-Hydroxy-3-methylvaleriansäure
Glyoxylharnstoff
β-Imidazolmilchsäure
2-Trifluormethyl-2-hydroxypropionsäure Hexahydromandelsäure
2-Hydroxyoctansäure
Arabinsäure
3-Phenylmilchsäure
Hydroxyphenylglycin
3-Hydroxymandelsäure
4-Hydroxymandelsäure
2-Hydroxynonansäure
3-Methoxymandelsäure
4-Methoxymandelsäure
3-(4-Hydroxyphenyl)milchsäure
Tartronsäure
β-Chlormilchsäure
1-Cyclopentanol-1-carbonsäure
1,2-Dihydroxycyclobutancarbonsäure
2-Ethyl-2-hydroxybuttersäure
α-Hydroxyisocapronsäure
α-Hydroxycapronsäure
2-Hydroxy-3,3-dimethylbuttersäure
Äpfelsäure
Hydroxytartronsäure
Gluconsäure
Milchsäureamid
N-Methylmilchsäureamid
N-Ethylmilchsäureamid
N,N-Dimethylmilchsäureamid
N-2-Hydxoxyethylmilchsäureamid
und deren Stereoisomeren und organischen oder
mineralischen Salzen sowie Solvaten.

7. Verfahren nach einem beliebigen der vorhergehenden Ansprüche, **dadurch gekennzeichnet, dass** die Verbindung nach Formel (I) aus den folgenden ausgewählt ist:
Glycolsäure
Oxalsäure
Milchsäure
2-Hydroxyisobuttersäure
2-Hydroxy-n-buttersäure
Glycerinsäure
2-Hydroxy-3-methylbuttersäure
2-Hydroxy-2-methylbuttersäure
2-Hydröxyvaleriansäure
4-Amino-2-hydroxybuttersäure
2-Methyläpfelsäure
Weinsäure
Citronensäure
2-Hydroxy-3-methylvaleriansäure
2-Hydroxyoctansäure
Arabinsäure
2-Hydroxynonansäure
Tartronsäure
2-Ethyl-2-hydroxybuttersäure
α-Hydroxyisocapronsäure
α-Hydroxycapronsäure
2-Hydroxy-3,3-dimethylbuttersäure
Äpfelsäure
Hydroxytartronsäure
Gluconsäure
und deren Stereoisomeren und organischen oder
mineralischen Salzen sowie Solvaten.

8. Verfahren nach einem beliebigen der vorhergehenden Ansprüche, **dadurch gekennzeichnet, dass** die Verbindung nach Formel (I) aus den folgenden ausgewählt ist:
Glycolsäure
Oxalsäure
Milchsäure
2-Hydroxyisobuttersäure
Äpfelsäure
Weinsäure
4-Amino-2-hydroxybuttersäure
Citronensäure
Gluconsäure
Tartronsäure
α-Hydroxycapronsäure
und deren Stereoisomeren und organischen oder
mineralischen Salzen sowie Solvaten.

## Claims

1. Process for relaxing the hair, comprising:
- a step of applying to the hair a hair-relaxing composition containing at least one α-hydroxy acid derivative, the pH of this composition being less than or equal to 7,
- and then a step of raising the temperature of the hair, using a heating means, to a temperature of between 140 and 250°C, an iron being applied by continuous movement from the root to the end, in one or more passes, the relaxing process being carried out without any reducing or lanthionization step,
the α-hydroxy acid derivative being chosen from
the compounds of formula (I): 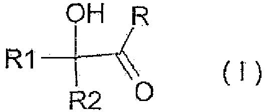 in which:
R1 = H, OH, NH2, CH2-COOH or a linear or branched C1-C4 alkyl,
R2 = H, COOH, CHOH-COOH, CF3, CH=CH2, NHCONH2, a linear, branched or cyclic C1-C8 alkyl optionally substituted with a radical chosen from OH, Cl, NH2, COOH, CF3 and SCH3;
a phenyl or benzyl optionally substituted with one OH or OCH3 radical; or alternatively the radical 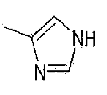
R1 and R2 may also together form an oxo radical (=O) or a cyclopropyl, cyclobutyl, hydroxycyclobutyl, cyclopentyl or cyclohexyl ring with the carbon atom that bears them, or alternatively a radical 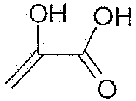
when R1 = H, R2 may also represent a (CHOH)2CH2OH or (CHOH)3CH2OH radical,
R = OH or NR3R4 with R3, R4 = H or a linear or branched C1-C4 alkyl optionally substituted with one or two OH radicals
and the stereoisomers, organic or mineral salts and solvates thereof.

2. Process according to Claim 1, **characterized in that** the temperature is raised using the heating means to a temperature of between 140°C and 220°C.

3. Process according to either of the preceding claims, **characterized in that** the composition is applied to wet hair.

4. Process according to any one of the preceding claims, **characterized in that** the hair is partially predried.

5. Process according to any one of the preceding claims, **characterized in that** the molar concentration of α-hydroxy acid derivative is between 2 and 8 M.

6. Process according to any one of the preceding claims, **characterized in that** the compound of formula (I) is chosen from:
glycolic acid
oxalic acid
lactic acid
1-hydroxy-1-cyclopropanecarboxylic acid
2-hydroxy-3-butenoic acid
2-hydroxyisobutyric acid
2-hydroxy-n-butyric acid
isoserine
glyceric acid
2-hydroxy-3-methylbutyric acid
2-hydroxy-2-methylbutyric acid
2-hydroxyvaleric acid
4-amino-2-hydroxybutyric acid
1-hydroxycyclohexanecarboxylic acid
dihydroxyfumaric acid
citramalic acid
tartaric acid
citric acid
2-hydroxy-4-(methylthio)butyric acid
mandelic acid
2-hydroxy-3-methylvaleric acid
glyoxylurea
β-imidazolelactic acid
2-trifluoromethyl-2-hydroxypropionic acid
hexahydromandelic acid
2-hydroxyoctanoic acid
arabic acid
3-phenylactic acid
hydroxyphenylglycine
3-hydroxymandelic acid
4-hydroxymandelic acid
2-hydroxynonanoic acid
3-methoxymandelic acid
4-methoxymandelic acid
3-(4-hydroxyphenyl)lactic acid
tartronic acid
β-chlorolactic acid
1-cyclopentanol-1-carboxylic acid
1,2-dihydroxycyclobutanecarboxylic acid
2-ethyl-2-hydroxybutric acid
α-hydroxyisocaproic acid
α-hydroxycaproic acid
2-hydroxy-3,3-dimethylbutyric acid
malic acid
hydroxytartronic acid
gluconic acid
lactamide
N-methyllactamide
N-ethyllactamide
N,N-dimethyllactamide
N-2-hydroxyethyllactamide
and the stereoisomers, organic or mineral salts
and solvates thereof.

7. Process according to any one of the preceding claims, **characterized in that** the compound of formula (I) is chosen from:
glycolic acid
oxalic acid
lactic acid
2-hydroxyisobutyric acid
2-hydroxy-n-butyric acid
glyceric acid
2-hydroxy-3-methylbutyric acid
2-hydroxy-2-methylbutyric acid
2-hydroxyvaleric acid
4-amino-2-hydroxybutyric acid
citramalic acid
tartaric acid
citric acid
2-hydroxy-3-methylvaleric acid
2-hydroxyoctanoic acid
arabic acid
2-hydroxynonanoic acid
tartronic acid
2-ethyl-2-hydroxybutric acid
α-hydroxyisocaproic acid
α-hydroxycaproic acid
2-hydroxy-3,3-dimethylbutyric acid
malic acid
hydroxytartronic acid
gluconic acid
and the stereoisomers, organic or mineral salts
and solvates thereof.

8. Process according to any one of the preceding claims, **characterized in that** the compound of formula (I) is chosen from:
glycolic acid
oxalic acid
lactic acid
2-hydroxyisobutyric acid
malic acid
tartaric acid
4-amino-2-hydroxybutyric acid
citric acid
gluconic acid
tartronic acid
α-hydroxycaproic acid
and the stereoisomers, organic or mineral salts
and solvates thereof.
